# EUROPÄISCHE PATENTSCHRIFT

(11) **EP 0 775 314 B1**
(45) Veröffentlichungstag und Bekanntmachung des Hinweises auf die Patenterteilung: **14.07.2004**
(21) Anmeldenummer: 95928414.2
(22) Anmeldetag: 04.08.1995
(51) Int. Cl.: G01N 33/543, C12Q 1/00, C12N 11/00, G01N 27/403

(54) **CHEMISCHE SENSOREN, INSBESONDERE BIOSENSOREN AUF SILICIUMBASIS**
CHEMICAL SENSORS, IN PARTICULAR SILICON-BASED BIOSENSORS
DETECTEURS CHIMIQUES, NOTAMMENT BIODETECTEURS, A BASE DE SILICIUM

(30) Priorität: 06.08.1994 DE 4427921
(43) Veröffentlichungstag der Anmeldung: 28.05.1997
(73) Patentinhaber: Forschungszentrum Jülich GmbH, 52425 Jülich (DE)
(72) Erfinder: SCHÖNING, Michael, Josef, D-52428 Jülich (DE); THUST, Marion, D-50939 Köln (DE); FROHNHOFF, Stephan, D-64295 Darmstadt (DE); BERGER, Michael, Götz, D-64287 Darmstadt (DE); ARENS-FISCHER, Rüdiger, D-45289 Essen (DE); KORDOS, Peter, D-52428 Jülich (DE); LÜTH, Hans, D-52076 Aachen (DE)
(86) Internationale Anmeldenummer: PCT/DE1995/001056
(87) Internationale Veröffentlichungsnummer: WO 1996/005512

(56) Entgegenhaltungen:
- WO-A-90/12092
- DE-A- 4 115 414
- PATENT ABSTRACTS OF JAPAN vol. 11 no. 52 (P-548) [2499] ,18.Februar 1987 & JP,A,61 218933 (MATUSHITA ELECTRIC INDUSTRIES) 29.September 1986, in der Anmeldung erwähnt
- JOURNAL OF APPLIED PHYSICS, Bd. 71, Nr. 8, 15.April 1992 WASHINGTON DC USA, Seiten r1-r22, R.L. SMITH ET AL. 'Porous silicon mechanisms' in der Anmeldung erwähnt

## Beschreibung

Die Erfindung bezieht sich auf chemische Sensoren, insbesondere Biosensoren, auf Siliciumbasis mit einem sensoraktiven Belag auf einem als Transduktor wirkenden Halbleitersubstrat.

Chemische Sensoren und insbesondere mit bioaktiven Komponenten arbeitende Biosensoren sind bekannt und in stürmischer Entwicklung begriffen.

Grundsätzlich umfassen solche Sensoren eine Oberflächenschicht mit sensoraktivem Material, die dem zu prüfenden Medium - insbesondere Flüssigkeit - ausgesetzt wird. Diese Schicht enthält das sensoraktive Material, das üblicherweise in einer z.B. aus PVC bestehenden Membran immobilisiert vorliegt. Das unter Einwirkung eines Analyten vom sensoraktiven Material gelieferte Signal wird von einem Transduktor-Element umgewandelt und mittels ggf. integrierter elektronischer Signalverarbeitung schließlich in registrierbarer Form erhalten.

Als Transduktoren sind insbesondere Halbleiterelektroden, Feldeffekttransistoren, potentiometrische und amperometrische Elektroden usw. im Gespräch.

Allen bekannten Sensoren gemeinsam ist die meist unzureichende Haftung der Sensormembran auf dem jeweiligen Grundbauelement, d.h. die zu untersuchende Meßlösung unterspült bzw. dringt im ungünstigsten Falle direkt in die Sensormembran ein und beschädigt diese irreversibel. Verbunden damit sind Stabilitäts- und Driftprobleme des Sensorausgangssignals. Weiterhin ist die Ausdiffusion, d.h. "das Ausbluten und das Auswaschen" der sensitiven Membrankomponenten in die Lösung hinein festzustellen. Solche Sensoren besitzen folglich nur eine begrenzte Lebensdauer. Nachteilig wirkt sich weiterhin die Tatsache aus, daß die Kontaktierung und Signalelektronik lediglich durch eine dünne Passivierungsschicht vom sensitiven Bereich getrennt und damit sehr störanfällig sind.

Ein Versuch zur Lösung dieses Problems wird von Knoll in der DE 41 15 414 A1 beschrieben: Danach werden anisotrope Ätzgruben als Containments in das Substratmaterial eingebracht, in denen die Sensormembran verankert werden soll. Die Containments können mit einem nichtleitenden Material ausgekleidet werden, zusätzlich können Ableiteelektroden abgeschieden werden. Diese vertikalen Containments besitzen eine sich zur Chip-Rückseite hin verjüngende Öffnung, die in unmittelbarem Kontakt mit der Meßlösung steht. Dadurch sollen die oben beschriebenen Nachteile umgangen werden. Insbesondere sollen gemäß dieser Technik sog. Sensor-Arrays mit verschiedenen Empfindlichkeiten für unterschiedliche Substanzen bzw. Ionensorten realisiert werden.

Bei dieser Sensorart wird durch die Containment-Bildung ein gewisser Schutz der ionenselektiven Membran vor Ausbluten und Ablösung erreicht. Diese Technologie ist jedoch nur schwerlich einfach, d.h. nur mit aufwendiger Lithographietechnik realisierbar.

Ziel der Erfindung ist daher eine Ausgestaltung von Sensoren der eingangs genannten Art, durch die sensoraktives Material in unterschiedlicher Form an unterschiedliche Typen von Transduktor/Elektronik des Sensors mit geringer Störanfälligkeit angelagert werden kann, wobei zusätzlich die Möglichkeit einer Stabilitätserhöhung sowie Empfindlichkeitssteigerung gegeben ist.

Dieses Ziel wird grundsätzlich durch chemische, insbesondere biologische Sensoren der im Anspruch 1 gekennzeichneten Art erreicht. Diese sind insbesondere in miniaturisierter Form zu realisieren.

Weitere Ausgestaltungen ergeben sich aus den Unteransprüchen.

Gemäß der Erfindung kann bei der Herstellung der porösen Schicht auf eine aufwendige Lithographie verzichtet werden. Die erzeugte dreidimensionale Schwammstruktur sorgt als Matrix für eine gute mechanische Verankerung und räumliche Vernetzung des sensoraktiven Materials im porösen Halbleitersubstrat. Man erreicht damit eine hohe physikalische (mechanische) und elektrochemische Stabilität unter Flüssigkeit. Damit bietet sich insbesondere der Einsatz im Durchflußbetrieb, z.B. als Detektor in FIA-Systemen, an.

Zwar ist durch Ätzbehandlung erzeugtes poröses Silicium an sich bereits längere Zeit bekannt und auch sein Einsatz in der Biosensortechnik gemäß der JP 61-218 932 A aus 1986 in Erwägung gezogen worden: Hier wird ein ISFET beschrieben, auf dessen Oberfläche zwischen Quelle und Senke eine Isolierschicht erzeugt und dann mit polykristallinem Silicium beschichtet wird, das durch anodische Behandlung in eine poröse Siliciumschicht umgewandelt als "Film" für eine biochemische Substanz wirken soll. Dieser Vorschlag hat ganz offensichtlich nicht anregend bezüglich der verbesserten Ausgestaltung von Biosensoren gewirkt und keinen Eingang in die Praxis gefunden, wie aus dem Vorschlag von Knoll zu entnehmen ist.

Von dem Inhalt dieser japanischen Offenlegungsschrift unterscheidet sich die vorliegende Erfindung dadurch, dass das als Transduktor wirkende Halbleitersubstrat unmittelbar einer Ätzbehandlung zur Erzeugung einer von der Oberfläche her in das Material vordringenden porösen Schwammstruktur unterworfen wird, deren Poren einen mittleren Porendurchmesser haben, der dem Eindringvermögen des sensoraktiven Materials angepasst ist.

Dabei werden schwammartige poröse Schichten mit Mesound/oder Makroporen, die - insbesondere unter Zwischenschaltung einer nichtleitenden Isolierschicht von geringer Dicke - das sensoraktive Material aufnehmen, dessen Effektivität pro Flächeneinheit (geometrische Fläche) damit erheblich gesteigert werden kann.

Die Meso- und Makroporen können durch Abzweigungen aufweisende Kanäle mit einem mittleren Porendurchmesser von 1 nm - 10 µm, insbesondere 10 - 1000 nm (Kanaldurchmesser) gebildet werden. Die schwammartige Schicht kann eine Dicke von 10 nm bis 100 µm aufweisen.

Art und Form des sensoraktiven Materials bestimmen natürlich die notwendige Schwammstruktur des porösen Siliciums, die durch Ätzbehandlung erzeugt wird.

Grundsätzlich erhält man je nach Dotierung unterschiedliche Porositätsprofile, wobei in n-Silicium mit Abzweigungen versehene tiefgreifende Ätzkanäle gebildet werden, deren mittlerer Porendurchmesser durch die gewählten Parameter von Vorbehandlung, Temperatur, Zusammensetzung des Elektrolyten, Anodisierungsstromdichte und -dauer sowie Nachbehandlung bestimmt wird. Durch Beleuchten während der anodischen Ätzbehandlung kann die Porenbildung beeinflußt werden, wobei insbesondere durch intermittierenden Lichteinfall ggf. erwünschte Durchmesserschwankungen im Porenkanal über die Länge hinweg erreicht werden können.

Die poröse Struktur, die sich beim Ätzen von p-Silicium sowie n⁺- bzw. p⁺-Silicium ergibt (mikroporös bzw. mit sog. "Fischgrätenverzweigungen") ist je nach Art des aufzubringenden sensoraktiven Materials einsetzbar. Eine detaillierte Zusammenstellung von Ätzbedingungen und Ätzergebnis findet sich bei R.L. Smith u. S.D. Collins im J. Appl. Phys. 71 R1 von 1992.

Aufgrund der Porendurchmesser der erzeugten Schwammstrukturen unterscheidet man zwischen Mikro- (<2 nm), Meso- (2 - 50 nm) und Makro (> 50 nm) porösem Silicium. Als für die erfindungsgemäße Ätzbehandlung zu variierende Prozeßparameter sind insbesondere zu nennen:
- die elektrochemische Vorbehandlung der Halbleitersubstrate (Flußsäure, organische und anorganische Lösungsmittel, Wasser, sowie deren Mischungen);
- die verwendeten Ätz- und Lösungsmittel (HF-Ethanol bzw. HF-Propanol-Gemisch);
- die Prozeßtemperatur (5 - 150°C);
- der Anodisierungsstrom (1 - 500 mA/cm²);
- die zusätzliche Beleuchtung während der Anodisierung (Wellenlänge λ = 200 - 800 nm, Intensität bzw. Leistung: 0,1 - 100 mW/cm², Standort der Beleuchtung: von der Probenoberseite oder -unterseite sowie die Frequenz der Beleuchtung f = 0,1 - 1000 Hz);
- die Nachbehandlung der porösen Schwammstruktur (Spülregime, z.B. in Ethanol / Temperatur / Lagerungsbedingungen, z.B. in N₂-Atmosphäre).

Die bevorzugt zu erzeugende nichtleitende Schicht auf den Porenwänden der Si-Schwammstruktur kann in an sich bekannter Weise aus (im einfachsten Fall) SiO₂ oder einer anderen dielektrischen Verbindung, wie Al₂O₃ oder Ta₂O₅ oder auch ZrO₂, Si₃N₄, Silikaten, Gläsern etc. - einzeln oder in Kombination - bestehen. Im Falle von SiO₂ wird dazu die vorhandene Si-Oberfläche der Schwammstruktur gezielt oxidiert. Dies läßt sich bevorzugt erreichen durch eine gleichmäßige thermische, anodische, chemische oder auch natürliche Oxidation. Die Schichtdicke der resultierenden nichtleitenden Schicht kann dabei, abhängig von der Porengröße, im Bereich von 1 - 100 nm variieren.

Im Falle von Al₂O₃ oder Ta₂O₅ wird zuerst das zugrundeliegende Metall (Al bzw. Ta) z.B. elektrochemisch, galvanisch oder aus der Gasphase abgeschieden und im Anschluß daran, wie oben für Si angegeben, in das entsprechende Oxid umgewandelt.

Die Abscheidung oxidischer, dielektrischer Verbindungen wie SiO₂, Al₂O₃, Ta₂O₅, ZrO₂, Si₃N₄ etc. durch herkömmliche PVD- und CVD-Verfahren direkt auf einem Siliciumsubstrat ist an sich bekannt (L. Bousse et al., Sensors and Actuators, B. 17 (1994), 157-164).

Sensoraktive Materialien, die erfindungsgemäß in bzw. an der so gebildeten porösen Struktur "verankert" werden, sind in großer Vielzahl bekannt. Beispielhaft werden daher nachfolgend unterschiedliche Systeme und die dafür zweckmäßige Porosität des Siliciums sowie die Art der Verankerung angegeben:

Neben der reinen Adsorption bzw. Chemisorption erscheint eine kovalente Anbindung von sensoraktivem Material an die poröse Struktur zweckmäßig, deren mittlerer Porendurchmesser zwischen 10 und 10³ nm gewählt wird. Die Porenoberfläche kann durch chemische Vorbehandlung oder Modifizierung, z.B. Silanisierung, aktiviert werden für die Anbindung von sensoraktivem Material. Ferner können sog. funktionelle Crosslinker (Spacermoleküle), wie z.B. Glutardialdehyd, an der Porengrenzfläche oder -wand verankert werden.

Eine komplette oder teilweise Vernetzung (z.B. nur im oberflächennahen Bereich) der in die Porenschicht eingebrachten Biomoleküle untereinander, z.B. mit Glutardialdehyd, kann für eine besonders stabile Integration des Biomaterials in der Schicht sorgen. Eine solche Vernetzung kann nach dem Einbringen der Biomoleküle in die poröse Schicht, z.B. durch Exposition mit einer Glutardialdehyd-gesättigten Atmosphäre erreicht werden. Der mittlere Porendurchmesser so zu behandelnder Schichten sollte ≳ 50 nm sein.

Biologische Strukturen, wie Enzyme, Proteine, Antikörper, Zellen, Organellen, Gewebsschnitte etc. können direkt oder mittels Geleinschluß, d.h. eingebettet in eine Trägermatrix aus Polymeren, wie Polyurethan, Polyacrylamid, Agar-Agar, Gelatine etc. ggf. räumlich vernetzt eingebracht werden, wobei je nach Größe des in die poröse Schwammstruktur einzubringenden Materials eine mittlere Porengröße im Bereich von 10 nm bis 100 µm, insbesondere ≳ 20 nm, gewählt wird.

Für die Verankerung von sensoraktivem Material in Form von Flüssigmembranen, die als "Membrancocktail", bestehend aus z.B.
- PVC
- Weichmacher
- ionenaktive Verbindung (Ionophor)
- Additive
verwendet werden, werden Porengrößen von zumindest 50 nm, vorzugsweise über 100 nm, vorgesehen.

Insbesondere für Chemosensoren eignen sich Glasschichten, die als flüssige Sol/Gelschicht + Benetzer in die Poren eingebracht werden - mit anschließender Temperung zur amorphen Glasschicht, die abhängig vom Ausgangscocktail für den Nachweis z.B. unterschiedlicher Alkaliionen brauchbar sind. Hier sind ebenso Porengrößen von ≳ 50 nm, insbesondere über 100 nm, zweckmäßig. Festkörperschichten aus galvanisch bzw. aus der Gasphase und auch elektrochemisch im porösen Material abgeschiedenem Metall, kombiniert mit Metallverbindungen (wie z.B. Ag/AgCl etc.), die für den Nachweis von Anionen nützlich sind, erweisen sich bezüglich der vorzusehenden Porengrößen als außerordentlich flexibel, wobei Porengrößen in der Gegend von 10 - 500 nm besonders zweckmäßig erscheinen.

Die gewählten sensoraktiven Substanzen können in bekannter Weise mit unterschiedlichen Sensortypen kombiniert angewandt werden: Nähere Einzelheiten sind der Literatur zu entnehmen, wie z.B. dem Buch von F. Scheller, F. Schubert, "Biosensoren", Akademie-Verl. Berlin, 1989.

Wie aus dem Vorstehenden folgt, ergibt sich eine große Vielfalt der Ausgestaltung der Erfindung. Einige wenige Beispiele werden nachfolgend anhand der beigefügten schematischen Zeichnungen wiedergegeben, die im einzelnen folgendes zeigen:
- Figur 1, 1a: den Querschnitt eines porösen (bio)chemischen Siliciumsensors mit Ausschnittsvergrößerung;
- Figur 2: einen kapazitiven Feldeffektsensor;
- Figur 3: einen Schnitt durch einen Feldeffekttransistor;
- Figur 4: eine potentiometrisch zu betreibende ionenselektive Elektrode (ISE) und
- Figur 5: die Anordnung eines Sensor-Arrays.

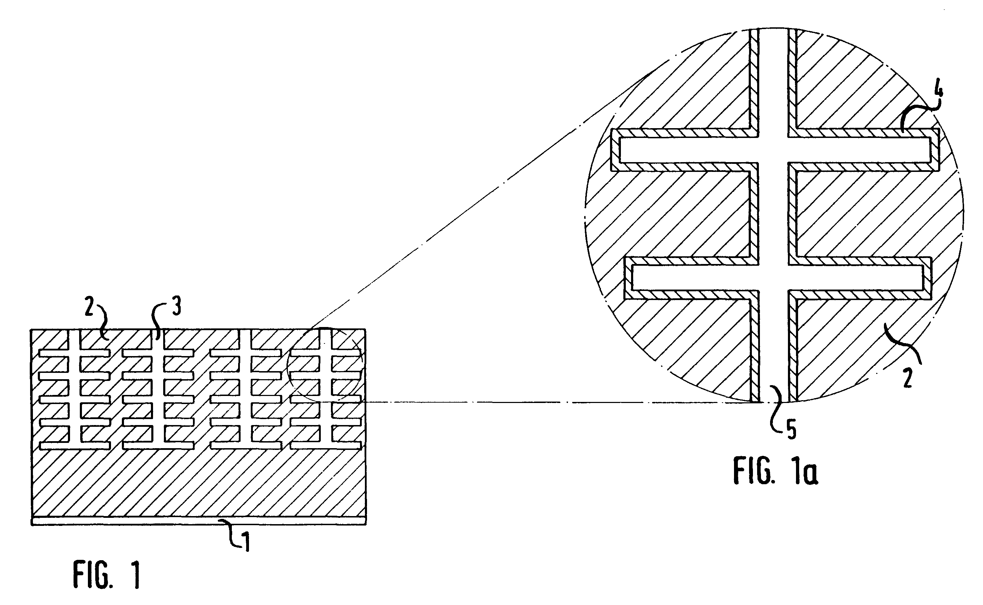

Figur 1 zeigt im Querschnitt den Schichtaufbau des porösen (bio-)chemischen Silicium-Sensors. Als Grundmaterial 2 wird dotiertes, ein- oder polykristallines Silicium verwendet. Die Dotierungskonzentration variiert im Bereich zwischen
1 x10¹⁴ - 1 x 10¹⁸/cm³ für n- bzw. p-Silicium und liegt bei > 1 x 10¹⁸/cm³ für n⁺- bzw. p⁺-Silicium. Auf der Unterseite von 2 befindet sich ein ohmscher Rückseitenkontakt 1, bestehend aus einer leitenden Schicht bzw. Schichtfolge, wie z.B. Al, Ti/Pt/Au, Cr/Sb/Au, oder ähnlicher leitender Verbindungen. Diese Kontaktschicht kann mittels herkömmlicher Beschichtungsverfahren, wie PVD-Abscheidung und Ionenimplantation oder auch durch elektrochemische Abscheideverfahren erzeugt werden.
Zur Herstellung der porösen Schichtstruktur wird das Grundsubstrat in eine chemisch inerte Probenzelle (z.B. Teflon) eingebaut und die Probe als Anode gegenüber einer in der Ätzlösung eingetauchten Kathode (z.B. aus Platin) geschaltet und eine Schwammstruktur 3 in folgender Weise erzeugt:
a) Wird als Ausgangsmaterial n-Silicium verwendet, so resultiert daraus, abhängig von der Beleuchtungsintensität und der Beleuchtungsseite eine unterschiedlich geartete Poren- und Kanalstruktur. Befindet sich die Quelle, z.B. eine Halogenlampe, auf der von 1 entgegengesetzten Seite, so bilden sich im Bulk des n-Siliciums vertikale, makroporöse Kanäle (Länge entspricht der Schichtdicke der porösen Struktur, Durchmesser: 0,1 - 10 µm) mit horizontalen, seitlichen Verzweigungen, sog. 'Sidebranches' vergleichbarer Dimension, 3, aus. Darüber bildet sich im Oberflächenbereich eine mikroporöse Schicht mit isotroper Porenstruktur (Durchmesser: < 5 nm), die in Kombination mit der makroporösen Schwammstruktur 3 genutzt oder nach Abschluß der Prozessierung mit einer NaOH-Lösung entfernt werden kann. Sowohl die Anodisierungsstromdichte (Zunahme der Porosität) als auch die Behandlungsdauer (Zunahme der Schichtdicke der porösen Siliciumstruktur) bestimmen entscheidend den Aufbau dieser makroporösen Schwammstruktur. Die Schichtdicke der sich parallel dazu bildenden mikroporösen Schicht wird im wesentlichen durch die Lichteindringtiefe, d.h. die Wellenlänge der Beleuchtungsquelle, bestimmt.
   Wird die Intensität der Beleuchtungsquelle weiterhin zeitabhängig verändert, d.h. wird während des Porenwachstums z.B. die Lichtquelle ein- und ausgeschaltet, so kann damit zusätzlich der Durchmesser der vertikalen Kanäle modifiziert werden. Während der Belichtungsphase bildet sich ein anderer Porendurchmesser aus als im Zeitraum der Dunkelphase. Es resultieren daraus 'wellenförmige, bauchartige' vertikale Poren- und Kanalstrukturen, die die mechanische Verankerung der anschließend abzuscheidenden Sensormembran unterstützen. Befindet sich die Beleuchtungsquelle auf der von 1 zugewandten Seite, bilden sich Makroporen in Form von vertikalen Kanalstrukturen ohne seitliche Verzweigungen aus. Abhängig von den Prozeßparametern variiert der Porendurchmesser dabei zwischen 100 nm und 10 µm. Die Länge dieser vertikalen Kanäle liegt im Bereich der Gesamtschichtdicke der porösen Schicht.
b) Im Gegensatz dazu bildet n⁺-dotiertes Silicium mesoporöse Schwammstrukturen (Porendurchmesser: 2 - 50 nm, Kanallänge entspricht der Schichtdicke der porösen Struktur) aus. Die horizontalen Verzweigungen verlaufen nicht streng orthogonal zu den vertikalen Kanälen. Die poröse Schichtstruktur ist vergleichbar mit einem 'Fischgrätenmuster', d.h. die Sidebranches sind in einer Neigung von < 45 Grad gegenüber den vertikalen Kanälen vorhanden.
c) Mikroporöse Schichtstrukturen (mittlerer Porendurchmesser < 2 nm) lassen sich vor allem mittels p-Silicium als Ausgangsmaterial realisieren. In diesem Fall bildet sich eine isotrope, homogen verteilte Porenanordnung aus. Der Porendurchmesser kann im oben angegebenen Bereich durch die Beleuchtung eingestellt werden, die Porosität ist anhand der Variation des Anodisierungsstromes einstellbar.
d) Wird als Grundmaterial p⁺-dotiertes Silicium eingesetzt, sind die damit erzielten Schwammstrukturen vergleichbar mit den Ergebnissen für n⁺-dotiertes Silicium. Die horizontalen Querverzweigungen entsprechen ganz analog in ihrer Dimension dem unter b) beschriebenen Aufbau. Ebenso sind die vertikalen Porendurchmesser sowie die Kanallänge in ihren geometrischen Ausdehnungen vergleichbar.

Die unter a) - d) variabel einstellbare Schwammstruktur eröffnet die Möglichkeit des gezielten Tailorings der sensoraktiven (bio-)chemischen Membran. Zu diesem Zweck wird die Schwammstruktur 3 (Ausschnittsdarstellung in Figur la) mit einem nichtleitenden Material 4 beschichtet. Die so gebildete poröse Schicht dient zur Aufnahme der sensoraktiven Komponenten 5.
Abhängig vom jeweiligen-Einsatzzweck kann das sensoraktive Material, wie bereits eingangs beschrieben, sowohl als ionenselektive Membran vorliegen als auch in Form von Biosensorelementen ausgestaltet sein, wobei nach Bedarf eine chemische Vorbehandlung (z.B. durch Silanisierung) vorgesehen werden kann, um eine gute direkte Anbindung des Sensormaterialsan bzw. in die poröse Schicht zu erzielen:
a) Die bekannte Herstellung von ionenselektiven Membranen kann auf dieselbe Art und Weise auch auf die poröse Schwammstruktur übertragen werden. Dazu wird das in einem Lösungsmittel enthaltene Membranmaterial (z.B. Ionophor, Weichmacher, PVC-Matrix) in die Schwammstruktur eingebracht, in der nach Abdampfen des Lösungsmittels eine Stabilisierung und Verfestigung stattfindet.
b) Alternativ besteht weiterhin die Möglichkeit, Biomoleküle in Form von Enzymen, Antikörpern-Antigenen, Gewebeschnitten, Organellen oder Rezeptoren als sensoraktive Membrankomponenten oder unmittelbar in der Schwammstruktur abzuscheiden. Dafür lassen sich sowohl gängige physikalische (z.B. Adsorption, Geleinschluß) als auch bekannte chemische Immobilisierungsverfahren (z.B. kovalente Bindung, Vernetzung) ausnutzen.

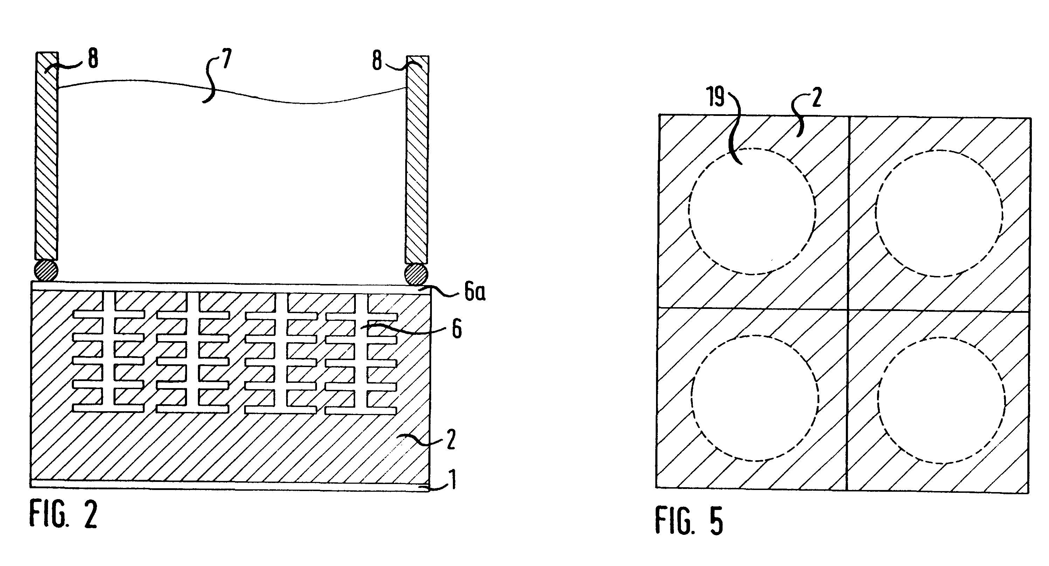

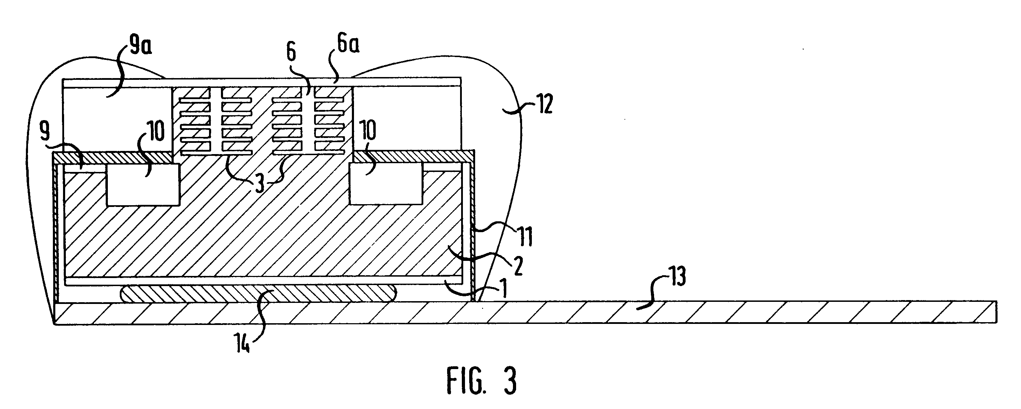

Figur 2 zeigt als Ausführungsbeispiel die Realisierung eines (bio-)chemischen porösen kapazitiven Feldeffektsensors. Der Schichtaufbau entspricht der in Figur 1 beschriebenen Anordnung. Das ggf. in Form einer Membran vorliegende sensoraktive Material 6 kann sowohl als Chemosensor als auch als Biosensor, abhängig von der jeweiligen Schichtzusammensetzung, funktionieren. Weiterhin besteht die Möglichkeit, das sensoraktive Material 6 in der Schwammstruktur und optional zusätzlich auf der Sensoroberfläche 6a abzuscheiden. Das poröse Sensorelement wird in einer geeigneten Meßzelle 8 (z.B. aus Teflon oder PMMA) gekapselt und direkt mit der Analytlösung 7 in Kontakt gebracht. Denkbar wäre allerdings auch eine Verkapselung, die dem Prinzipaufbau der in Figur 3 weiter unten aufgeführten Beschreibung entspricht.
Zur Herstellung der elektrischen Verbindung zwischen der Analytlösung 7 und dem metallischen Substratkontakt 1 ohmscher Rückenseitenkontakt kann z.B. eine potentialkonstante, kommerziell erhältliche Referenzelektrode, die in 7 eingebracht und mit 1 verbunden ist, eingesetzt werden. Anstelle dieser ist es jedoch auch möglich, ein gleichartiges, nicht sensitives poröses Sensorelement als Referenzelement zu verwenden. In diesem Fall besteht der Vorteil der Anordnung vor allem in der Miniaturisierbarkeit, die in der Regel durch die Größe der Referenzelektrode limitiert ist, und in der verringerten Auswirkung äußerer Einflüsse, wie z.B. unterschiedlicher Temperaturkoeffizienten von Sensorelement und Referenzelektrode.

In Figur 3 ist der Aufbau eines porösen
(bio-)chemischen Feldeffekttransistors ausgeführt. Das verwendete Grundmaterial entspricht dem des oben beschriebenen kapazitiven Siliciumsensors. Abhängig von der Dotierung des Grundmaterials 2 besitzen die beiden Taschen, Source und Drain, 10 die entgegengesetzte Dotierung. Ist das Grundmaterial n-dotiert, sind Source und Drain p-dotiert und umgekehrt. Diese sind über eine metallische Kontaktierung 11 (z.B. Ti/Al, Ti/Pd/Au, o.ä. leitende Materialien) mit einem festen Träger, z.B. einem Leiterplattensubstrat Grundträger 13, verbunden. Die Metallische Kontaktierung 11 ist durch eine Isolatorschicht bzw. -schichtfolge 9 und 9a aus SiO₂ oder SiO₂/Si₃N₄ oder SiO₂/Al₂O₃ oder SiO₂/Ta₂O₅ o.ä. vom Grundsubstrat isoliert aufgebracht. Die Herstellung solcher Feldeffekttransistoren ist aus der Literatur bekannt (K. Horninger, Integrierte MOS-Schaltungen, Springer-Verlag (1987) Heidelberg).
Neu an dieser Anordnung ist die Nutzung des Gatebereiches zwischen den beiden Taschen 10 in Form eines porösen Silicium-Gates. Dazu muß während der eigentlichen Prozessierung, d.h. unmittelbar nach der Dotierung der beiden Taschen 10, ein wie in der Abbildung dargestelltes 'hochstehendes' Gate, z.B. über einen zusätzlichen Photolithographie- und Ätzschritt, erzeugt werden. Alternativ ließe sich ein solches Gate auch mittels in der Halbleitertechnologie eingesetzter Verfahren, wie z.B. selektive Epitaxie u.ä. realisieren.
Die Erzeugung der Schwammstruktur 3 im Siliciumausgangsmaterial bzw. die im Anschluß darauf folgende Abscheidung des sensoraktiven Materials 6 erfolgt in ganz analoger Weise wie für Figur 1 beschrieben. Der Substratkontakt 1 zum Grundträger 13 wird durch eine leitende Klebeverbindung 14, z.B. durch Leitsilber realisiert. Das Sensorbauelement wird mit einer Schutzschicht 12 aus z.B. Epoxidharz oder anderen Vergußmaterialien so gegenüber der Meßumgebung geschützt, daß lediglich der sensoraktive Gatebereich mit der Analytlösung in Kontakt steht. Die Verkapselung könnte jedoch auch mittels eines Einbaus in eine feste Probenzelle, wie in Figur 2 beschrieben, erfolgen. Die Möglichkeit, ähnlich wie beim kapazitiven porösen (bio-)chemischen Sensor anstelle einer äußeren Referenzelektrode direkt ein nicht sensitives Sensorelement einzusetzen, ist auch mit diesem Aufbau in Form eines Referenztransistors lösbar.

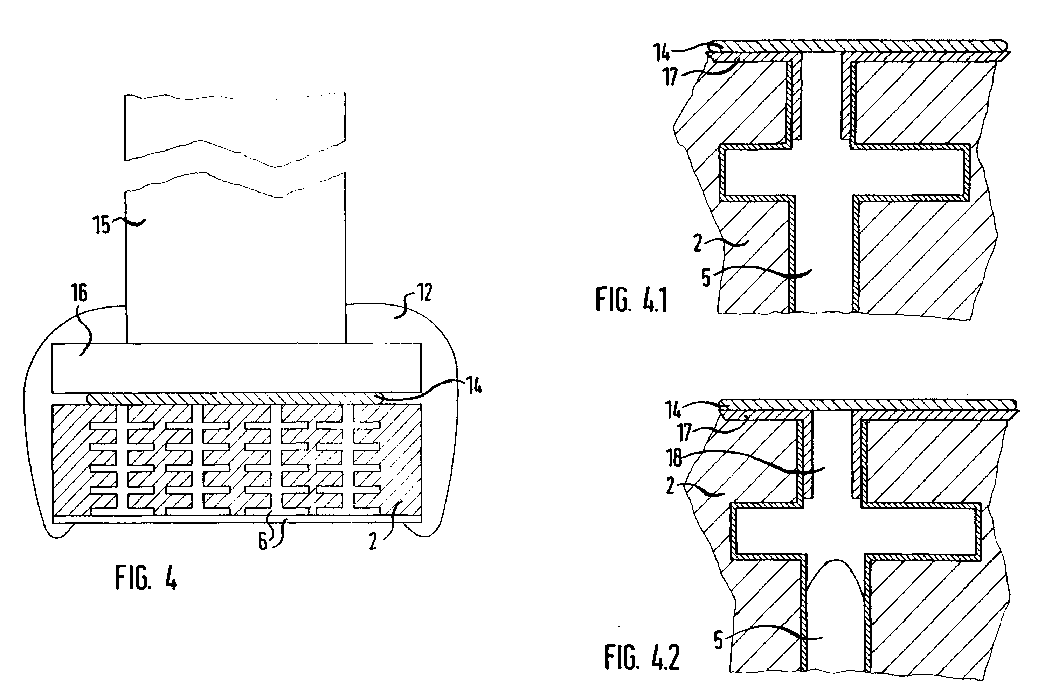

Ein Ausführungsbeispiel in Form einer porösen, potentiometrischen (bio-)chemischen 'Ionenselektiven Elektrode' (ISE) ist aus Figur 4 zu ersehen. Die Herstellung der porösen Schwammstruktur und deren Auskleidung mit einem nichtleitenden Material erfolgt in analoger Weise wie unter Figur 1 beschrieben. Im Anschluß daran wird das Grundsubstrat Grundmaterial 2 von der Probenrückseite von 1 aus naßchemisch z.B. mittels eines HF/Wasser-Gemisches bis in den Bereich der Schwammstruktur abgeätzt, so daß diese auf der Probenrückseite freiliegt.

Bevor die sensoraktiven Material 6 als Fest- bzw. Flüssigkeitselektrolyt in ganz analoger Weise, wie unter Figur 1 bechrieben, in die Schwammstruktur eingebracht werden, muß die metallische Ableitung, wie in Figur 4.1 bzw. Figur 4.2 aufgezeigt, realisiert werden. Dazu wird in Figur 4.1 ein Metallfilm 17, beispielsweise aus Ag mittels herkömmlicher PVD-Verfahren (z.B. durch Aufdampfen) in der Porenstruktur abgeschieden. Die Chloridisierung dieser Ag-Schicht mittels bekannter elektrochemischer Verfahren erfolgt in direktem Anschluß. Ein zusätzlicher Innenelektrolyt 18 wird in Figur 4.2 eingebracht. Dieser besteht in der Regel aus einer hochmolaren Salzlösung, z.B. gesättigter KCl-Lösung, der in einer festen organischen Matrix, z.B. Gelatine, nach Abdampfen des Lösungsmittels als Innenelektrolyt zurückbleibt.
Die so prozessierte Halbleiterstruktur wird mittels einer leitenden Klebeverbindung 14, z.B. Leitsilber auf einem Träger 16 (Figur 4) mit elektrischer Kontaktierung, z.B. aus Glas, Kunststoff, Silicium oder Keramik fixiert. Dadurch wird die dünne, poröse Siliciumstruktur auf dem Träger 16 stabilisiert. Die Prozessierung der sensoraktiven Schicht erfolgt wie bereits unter Figur 1 ausgeführt. Die fertiggestellte poröse ISE wird auf eine Halterung 15 aus lösungsbeständigem Material mit elektrischer Kontaktierung, z.B. Teflon oder Kunststoff, aufgebracht. Das Sensorbauelement wird mit einer Schutzschicht 12 aus z.B. Epoxidharz oder anderen Vergußmaterialien so gegenüber der Meßumgebung geschützt, daß lediglich der sensoraktive Bereich der porösen ISE mit der Analytlösung in Kontakt steht.

Figur 5 zeigt die Anordnung eines (bio-)chemischen porösen Halbleitersensors als Multi-Sensor in Form einer Sensorarray-Anordnung. Hier dargestellt sind unterschiedlich sensitive, poröse Sensoren 19 innerhalb eines Siliciumsubstrates Grundmaterial 2. Dazu wird im ersten Schritt über eine einfache photolithographische Strukturierung die Anzahl der Sensorelemente (z.B. vier variable Sensoren) festgelegt. Im Anschluß daran werden, der Anzahl entsprechend, die einzelnen porösen Schwammstrukturen erzeugt und mit den oben aufgeführten Prozeßschritten zu Sensorelementen ausgebaut. Abhängig vom jeweiligen Verwendungszweck lassen sich hiermit die in Figur 2, Figur 3, Figur 4 beschriebenen Sensorelemente realisieren. Die Sensorelemente sind ganz analog in entsprechender Art und Weise in einer Probenzelle gekapselt bzw. in ein festes Gehäuse eingebracht.

## Patentansprüche

1. Chemische Sensoren, insbesondere Biosensoren, auf Siliciumbasis mit einem sensoraktiven Belag auf einem als Transduktor wirkenden Halbleitersubstrat, **gekennzeichnet durch** eine **durch** Ätzbehandlung der Substratoberfläche erzeugte, von der Oberfläche her vordringende, eine Schwammstruktur aufweisende poröse Schicht mit in die Poren eingreifendem sensoraktiven Material, deren Öffnungsweite dem Eindringvermögen des sensoraktiven Materials angepaßt ist, wobei die schwammartige poröse Schicht Meso- und/ oder Makroporen aufweist und eine nichtleitende Grenzschicht auf den Porenwänden vorgesehen ist.

2. Sensoren nach Anspruch 1,
**gekennzeichnet durch**
eine zwischen den Oberflächen des porösen Materials und dem sensoraktiven Material befindliche Grenzschicht aus Siliciumdioxid und/oder Aluminiumoxid, Tantalpentoxid bzw. Siliciumnitrid.

3. Sensoren nach Anspruch 1 oder 2,
**gekennzeichnet durch**
eine Dicke der nichtleitenden Grenzschicht von 1 - 100 nm.

4. Sensoren nach einem der vorhergehenden Ansprüche,
**dadurch gekennzeichnet,**
**dass** die Poren durch Abzweigungen aufweisende Kanäle mit einem mittleren Porendurchmesser von 1 nm - 10 µm, insbesondere 10 - 1000 nm (Kanaldurchmesser) gebildet werden.

5. Sensoren nach einem der vorangehenden Ansprüche,
**gekennzeichnet durch**
eine Dicke der schwammartigen Schicht von 10 nm - 100 µm.

6. Sensoren nach einem der vorangehenden Ansprüche,
**gekennzeichnet durch**
adsorptiv in bzw. an der Schwammstruktur angelagertes sensoraktives Material.

7. Sensoren nach einem der Ansprüche 1 bis 5,
**gekennzeichnet durch**
eine kovalente Bindung des sensoraktiven Materials an die poröse Schichtstruktur.

8. Sensoren nach einem der Ansprüche 1 bis 5,
**gekennzeichnet durch**
eine Vernetzung der Biomoleküle des in die Poren eingreifenden sensoraktiven Materials.

9. Sensoren nach einem der Ansprüche 1 bis 5,
**dadurch gekennzeichnet,**
**daß** das sensoraktive Material innerhalb eines Schichtbildners verteilt vorliegt.

10. Sensoren nach Anspruch 9,
**dadurch gekennzeichnet,**
**daß** das sensoraktive Material in einer Glas- oder Festkörper- oder Kunststoff- bzw. Polymermembran verteilt vorliegt.

11. Sensoren nach einem der vorangehenden Ansprüche, ausgebildet als ionenselektive Elektroden.

12. Sensoren nach einem der Ansprüche 1 bis 10, ausgebildet als kapazitive Feldeffektstrukturen.

13. Sensoren nach einem der Ansprüche 1 bis 10, ausgebildet als ionenselektive Feldeffekttransistoren.

14. Sensoren nach einem der vorangehenden Ansprüche, gebildet als Array unterschiedlich sensitiver Elemente.

15. Sensoren nach Anspruch 14,
**gekennzeichnet durch**
ein nicht-sensitives Referenzelement als Array-Komponente.

## Claims

1. Chemical sensors, in particular biosensors, based on silicon with a sensor-active coating on a semiconductor substrate acting as a transductor, **characterized by** a porous layer produced by etching treatment of the substrate surface, penetrating from the surface and having a sponge structure, with sensor-active material engaging into the pores, the opening width of which is adapted to the penetration capability of the sensor-active material, the spongy porous layer having mesopores and/or macropores and a non-conductive boundary layer being provided on the pore walls.

2. Sensors according to Claim 1,
**characterized by**
a boundary layer made of silicon dioxide and/or aluminium oxide, tantalum pentoxide or silicon nitride which is situated between the surfaces of the porous material and the sensor-active material.

3. Sensors according to Claim 1 or 2,
**characterized by**
a thickness of the non-conductive boundary layer of 1 - 100 nm.

4. Sensors according to one of the preceding claims,
**characterized**
**in that** the pores are formed by channels having branchings with an average pore diameter of 1 nm - 10 µm, in particular 10 - 1000 nm (channel diameter).

5. Sensors according to one of the preceding claims,
**characterized by**
a thickness of the spongy layer of 10 nm - 100 µm.

6. Sensors according to one of the preceding claims,
**characterized by**
sensor-active material added adsorptively in or at the sponge structure.

7. Sensors according to one of Claims 1 to 5,
**characterized by**
a covalent bond of the sensor-active material to the porous layer structure.

8. Sensors according to one of Claims 1 to 5,
**characterized by**
a crosslinking of the biomolecules of the sensor-active material engaging into the pores.

9. Sensors according to one of Claims 1 to 5,
**characterized**
**in that** the sensor-active material is present in a manner distributed within a layer former.

10. Sensors according to Claim 9,
**characterized**
**in that** the sensor-active material is present in a manner distributed in a glass or solid or plastic or polymer membrane.

11. Sensors according to one of the preceding claims, formed as ion-selective electrodes.

12. Sensors according to one of Claims 1 to 10, formed as capacitive field-effect structures.

13. Sensors according to one of Claims 1 to 10, formed as ion-selective field-effect transistors.

14. Sensors according to one of the preceding claims, formed as an array of elements of different sensitivity.

15. Sensors according to Claim 14,
**characterized by**
a non-sensitive reference element as an array component.

## Revendications

1. Détecteurs chimiques, notamment biodétecteurs, à base de silicium ayant une couche active du point de vue du détecteur sur un substrat semi-conducteur agissant en tant que transducteur, **caractérisé par** une couche poreuse produite par un traitement d'attaque de la surface du substrat, présentant une structure spongieuse, sortant de la surface avec de la substance active du point de vue du détecteur, pénétrant dans les pores et dont la largeur d'ouverture est adaptée à la puissance de pénétration de la substance active du point de vue du détecteur, la couche poreuse de type éponge ayant des mésopores et/ou des macropores et il est prévu une couche limite non-conductrice sur les parois des pores.

2. Détecteurs suivant la revendication 1, **caractérisés par** une couche limite se trouvant entre la surface de la matière poreuse et la matière active du point de vue du détecteur, qui est en dioxyde de silicium et/ou en oxyde d'aluminium, en pentoxyde de tantale ou en nitrure de silicium.

3. Détecteurs suivant la revendication 1, **caractérisés en ce que** par une épaisseur de la couche limite non-conductrice de 1 à 100 nm.

4. Détecteurs suivant l'une des revendications précédentes, **caractérisé en ce que** les pores sont formés par des canaux présentant des dérivations, d'un diamètre moyen de pore de 1nm à 10 µm, notamment de 10 à 1000 nm (diamètre de canal).

5. Détecteurs suivant l'une des revendications précédentes, **caractérisés par** une épaisseur de la couche de type éponge de 10 nm à 100 nm.

6. Détecteurs suivant l'une des revendications précédentes, **caractérisés par** de la matière active du point de vue du détecteur emmagasinée par adsorption dans ou sur la structure spongieuse.

7. Détecteurs suivant l'une des revendications 1 à 5, **caractérisés par** une liaison covalente de la matière active du point de vue du détecteur sur la structure en couche poreuse.

8. Détecteurs suivant l'une des revendications 1 à 5, **caractérisés par** une réticulation des biomolécules de la matière active du point de vue du détecteur pénétrant dans les pores.

9. Détecteurs suivant l'une des revendications 1 à 5, **caractérisés en ce que** la matière active du point de vue du détecteur est répartie au sein d'un agent filmogène.

10. Détecteurs suivant la revendication 9, **caractérisés en ce que** la matière active du point de vue du détecteur est répartie dans un corps en verre ou solide ou dans une membrane en matière plastique ou en polymère.

11. Détecteurs suivant l'une des revendications précédentes, constitués sous la forme d'une électrode à sélection d'ions.

12. Détecteurs suivant l'une des revendications 1 à 10, constitués en structures capacitives à effet de champ.

13. Détecteurs suivant l'une des revendications 1 à 10, constitués en transistors à effet de champ sélectifs en ions.

14. Détecteurs suivant l'une des revendications précédentes, sous la forme d'un réseau d'éléments de sensibilité différente.

15. Détecteurs suivant la revendication 14, **caractérisés par** un élément de référence non sensible sous la forme de composants du réseau.
